# EUROPEAN PATENT SPECIFICATION

(11) **EP 4 051 192 B1**
(45) Date of publication and mention of the grant of the patent: **30.07.2025**
(21) Application number: 20819924.0
(22) Date of filing: 02.11.2020
(51) Int. Cl.: A61F 9/00, A61F 2/00, A61F 2/02, A61P 9/00

(54) **ANALYTE DIFFUSIVE IMPLANTABLE DEVICE**
IMPLANTIERBARE ANALYSE-DIFFUSIONSVORRICHTUNG
DISPOSITIF IMPLANTABLE DE DIFFUSION D'ANALYTE

(30) Priority: 01.11.2019 US 201962929619 P; 04.11.2019 US 201962930504 P
(43) Date of publication of application: 07.09.2022
(73) Proprietor: Neurotech USA, Inc., Cumberland, RI 02864 (US)
(72) Inventor: KAUPER, Konrad A., Sutton, Massachusetts 01590 (US); MILLS, John F., Wakefield, Rhode Island 02879 (US); SHERMAN, Sandy, Fairhaven, Massachusetts 02719 (US); NYSTUEN, Arne M., Brookline, Massachusetts 02445 (US)
(74) Representative: Haseltine Lake Kempner LLP
(86) International application number: PCT/US2020/058589
(87) International publication number: WO 2021/087481

(56) References cited:
- US-A- 4 892 538
- US-A- 5 653 688
- US-A- 5 904 144
- US-A1- 2008 286 323
- US-A1- 2011 236 457
- US-A1- 2015 073 381

## Description

### RELATED APPLICATIONS

The present disclosure claims benefit of and priority to U.S. provisional application no. 62/929,619, filed November 1, 2019, and U.S. provisional application no. 62/930,504, filed November 4, 2019.

### BACKGROUND

Transporting a bioartificial organ (BAO), or other implantable medical device, especially those of smaller size (e.g., finger-tip size or smaller), can be quite challenging. Given their size, improper handling can lead to damage or sterility-breach of the devices, which, if detected, are discarded, or if undetected may result in potential risk to patients. Moreover, such devices, if required to be filled with a substance, or, multiple steps in a manufacturing process, also requires delicate handling, as well as associated structure to aid in filling and/or manufacturing steps. Improper handling can lead to, yet again, potential risk to patients. This is particularly problematic in semi-automated or automated manufacture of a BAG for treatment of retinal disorders. Document US-A-2015/073381 discloses a medical device system comprising: an analyte diffusive implantable device (ADID) which comprises: a cavity; a cell-scaffolding material (CSM) arranged within the cavity and configured to hold or otherwise retain a plurality of living cells; and a porous membrane at least partially enclosing the cavity, wherein at least a portion of the membrane is adhered to the CSM by a high durometer adhesive which creates a hemispheric seal at both ends of the membrane; and a filling port having: docking means at a first end, and a micro-diameter tube (MDT) extending from a second end, wherein the MDT is sealed at a distal end to the filling port and at a proximal end to the cavity of the ADID, so as to establish an integral, cell-filling pathway there-within.

### SUMMARY OF SOME OF THE EMBODIMENTS

In some embodiments of the present disclosure, a medical device system is provided and includes a medical device, and/or, in some embodiments, an analyte diffusive implantable device (ADID) having a cavity, a cell-scaffolding material (CSM) arranged within the cavity and configured to hold or otherwise retain a plurality of living cells, and a porous membrane at least partially enclosing the cavity, wherein at least a portion of the membrane is adhered to the CSM by a high durometer adhesive which creates a hemispheric seal at both ends of the membrane. The system also includes a filling port having docking means at a first end, and a micro-diameter tube (MDT) extending from a second end. The MDT is sealed at a distal end to the filling port and at a proximal end to the cavity of the ADID, so as to establish an integral, cell-filling pathway there-within.

The present invention relates to a device as set forth in the appended claims.

In such embodiments, at least one of the following additional features, functionality, structure, steps, and/or clarifications (and in some embodiments, a plurality of, and in some embodiments, all of) can be included, leading to yet further embodiments:
- the filling port corresponds to (or includes, comprises) a temporary filling port;
- at least one anchor arranged on at least one end of the device;
   o the anchor is configured to affix the device to tissue;
   o the anchor is configured to be held by a clip, where the clip can be configured to enable packaging and/or transfer of the device;
   and
- a clip, which can include at least one of (or a plurality of, or all of):
   o a first jaw having a corresponding first distal jaw end;
   o a second jaw having a corresponding second distal jaw end configured for movement relative to the first jaw end;
   o a first jaw extension integral with the first jaw;
   o a second jaw extension integral with the second jaw; and/or
   o at least two proximal extensions integral with the first jaw;
   o the first extension can include any or all of a first projection, a first receiving area and a second receiving area;
   o the second extension can include a flexible projection;
   o the first receiving area can be configured to receive an end of the flexible projection;
   o the second receiving area can be configured to receive the second jaw extension and move therein; and
   o the first distal end of the first jaw can be closed with respect to the second distal end of the second jaw in a resting position.

In some embodiments, an analyte diffusive system is provided and includes a medical device, and/or, in some embodiments an analyte diffusive implantable device (ADID) having a cavity, a cell-scaffolding material (CSM) arranged within the cavity and configured to hold or otherwise retain a plurality of living cells, a porous membrane at least partially enclosing the cavity, wherein at least a portion of the membrane is adhered to the CSM by a high durometer adhesive which creates a hemispheric seal at both ends of the membrane, and a filling port configured for connection to an end of the device, wherein the port is configured to be in communication with the cavity and sealed thereto, to provide access to the cavity to enable cells to be received in the cavity, and as a polymer molded structure so as to allow an injection system to seal to thereto.

In such embodiments, at least one of the following additional features, functionality, structure, steps, and/or clarifications (and in some embodiments, a plurality of, and in some embodiments, all of) can be included, leading to yet further embodiments:
- a micro-diameter tube (which can also be, in some embodiments, a "tube" of larger size) having an inner micron-sized lumen (for example) configured to flow cells from an injection system into the cavity;
   o a first end of the tube is configured to connect to an end of the implantable device,
   o a second end of the tube is configured to connect to the port assembly, and
   o cells are delivered from the injection system into the cavity from the port assembly and through the tube;
- the tube can include a wall thickness and/or material configured to maintain a diameter geometry for delivery of cells through the tube and maintain the diameter geometry at a fill pressure of 0 - 150 psi (for example);
- the tube is sealed to the port assembly with a first adhesive;
- the tube is sealed within the CSM and membrane with a second adhesive, having properties distinct from properties associated with a first adhesive;
- the tube is configured for preferential release from a first adhesive;
- a force required to preferentially release the sealed filling port from within the device requires between 1-50 Newtons (for example);
- filling of cells within the cavity is selected from the group consisting of: capillary action, positive displacement, vacuum, servo driven, peristaltic action, and combinations thereof;
- a clip;
   o the clip is configured to enable packaging and/or transfer of the device, which can include at least one of (and in some embodiments a plurality of, and in some embodiments all of):
      ▪ a proximal end and a distal end;
      ▪ a first jaw having a corresponding first distal jaw end;
      ▪ a second jaw having a corresponding second distal jaw end configured for movement relative to the first jaw end;
      ▪ a first jaw extension integral with the first jaw;
      ▪ a second jaw extension integral with the second jaw; and/or
      ▪ at least two proximal extensions integral with the first jaw;
   o the first extension includes a first projection, a first receiving area and a second receiving area;
   o the second extension includes a flexible projection;
   o the first receiving area being configured to receive an end of the flexible projection;
   o the second receiving area being configured to receive the second jaw extension and move therein; and
   o the first distal end of the first jaw is closed with respect to the second distal end of the second jaw in a resting position.

In such embodiments, a cell transferring method for transferring cells into an analyte diffusive device is provided and includes providing the analyte diffusive system according to any of the disclosed embodiments, sealing the tube with the device and the port assembly, transferring cells into the device via the port assembly and tube using the injection system, wherein the transferred cells are stored on the CSM within cavity, and removing at least one of the port assembly and tube from the device.

The method of using the device does not form part of the invention.

In some embodiments, an aseptic transferring method for transferring or otherwise moving an implantable device is provided and includes providing an analyte diffusive implantable device (ADID), providing a clip, holding the device with the clip, wherein the clip attaches to the device either at one end of the device or via an anchor attached to the end of the device, and moving the device via the clip through automated manufacturing unit operations to fill the device.

In some embodiments, an aseptic method to maintain orientation of device during storage and use for surgical implant is provided and includes providing an analyte diffusive system including an analyte diffusive implantable device (ADID), providing a clip, holding the ADID with the clip, where the clip attaches to the ADID either at one end of the ADID or via an anchor attached to the end of the ADID and orients the ADID vertically and centrally in a packaging system containing cell nutrient media, and optionally aseptic transferring of the ADID from the packing system to a sclerotomy incision and facilitating positioning of the ADID and release of the ADID into the incision and vitreous cavity.

These and other embodiments, objects and advantages will become even more evident with reference to the concurrently filed figures, a brief description of which is set out below, and following detailed description of at least some of the embodiments.

### BRIEF DESCRIPTION OF THE FIGURES

**Figure 1** illustrates an assembled view of a retaining system for filling, holding, and/or retaining an biomedical implant, according to some embodiments of the disclosure.
**Figure 2** illustrates an enlarged, side view of an implant and clip for the system of Figure 1, according to some embodiments of the disclosure.
**Figure 3A** illustrates an enlarged, side, cross-sectional view of an implant and anchor of the assembly, according to some embodiments of the disclosure.
**Figure 3B** illustrates an assembled view of a retaining system for filling, holding, and/or retaining a biomedical implant, according to some embodiments of the disclosure.
**Figure 4** illustrates a perspective view of an anchor for the implant, for the assembly, according to some embodiments of the disclosure.
**Figure 5A** illustrates a side view of a clip of the assembly, according to some embodiments of the disclosure.
**Figure 5B** illustrates a perspective view of the clip of Figure 5A, according to some embodiments of the disclosure.

### DETAILED DESCRIPTION OF AT LEAST SOME OF THE EMBODIMENTS

In the present description, 1 psi is equivalent to 6894.76 Pascals.

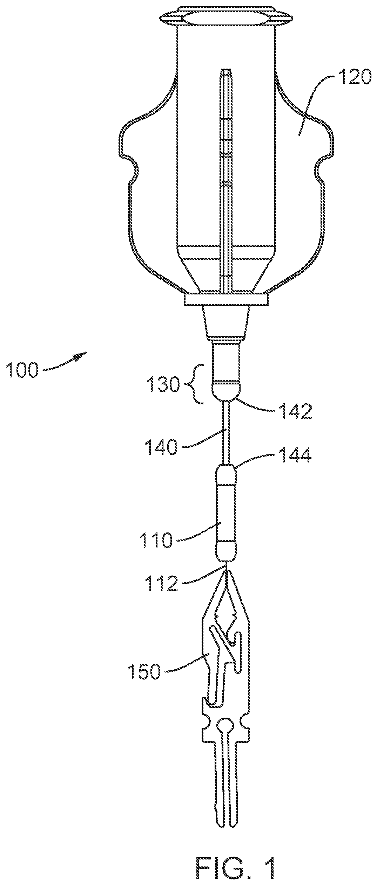

**Figures 1-5B** illustrate embodiments of the present disclosure. Figure 1 illustrates a first-side view of a medical device system, according to some embodiments, and includes an analyte diffusive implantable device (ADID) 110, which includes a cavity, and a cell-scaffolding material (CSM) arranged within the cavity and configured to hold or otherwise retain a plurality of living cells engineered to secrete a therapeutic protein-based drug (according to some embodiments). The system can also include a porous hollow fiber membrane at least partially enclosing the cavity (*see* U.S. patent nos. 7115257, 6361771, 9265814, 10195140.

At least a portion of the membrane is adhered to the CSM by a high durometer adhesive (for example, with respect to some embodiments) which can create a preferably hemispheric seal at one, and preferably, both ends of the membrane. Also included is a filling port 120 (which in some embodiments can be referred to as a filling port assembly), having a docking means 130, which may also be referred to a port assembly, at a first end, and a micro-diameter tube (MDT) 140 extending from a second end. The MDT is preferably sealed (and preferably, hermetically) at a distal end 142 to the filling port 120, and at a proximal end 144 to the cavity of the ADID 110, so as to establish an integral, cell-filling pathway there-within.

In some embodiments, the ADID/system 110 includes at least one anchor 112 arranged on at least one end of the device, where the anchor can be configured to affix the device 110 to tissue (for example). The anchor 112 can also be configured to at least one of be received and held by a clip- device 150 ("clip"). The clip 150 can be configured to enable packaging and/or transfer of the ADID.

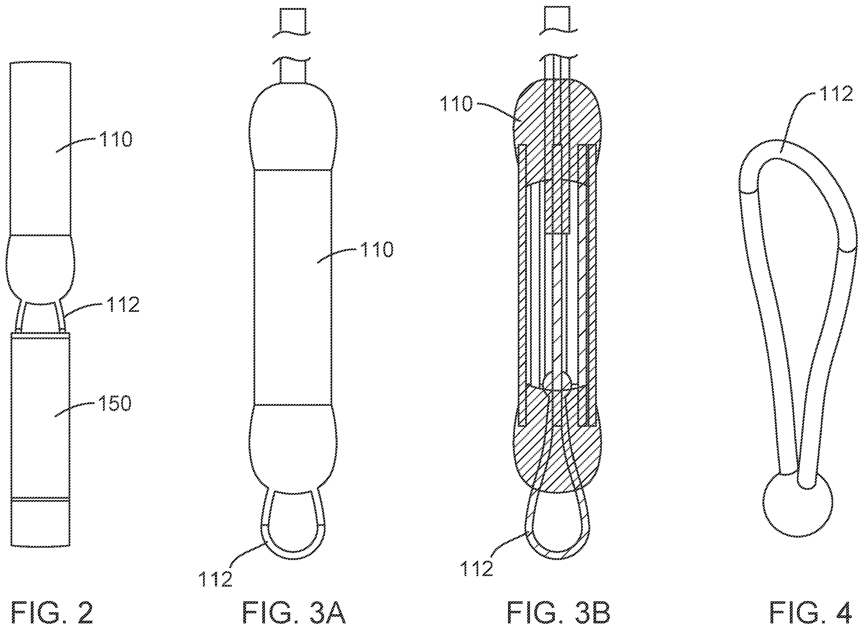

In such embodiments, the filling port 120 corresponds to (or includes, comprises) a temporary filling port, such that, it is only used to fill the ADID, and then is of no further use and can be removed/discarded/reused. The at least one anchor (in some embodiments), can be a plurality of anchor, and arranged on at least one end of the device is configured to affix the device to tissue. **Figures 2-4** illustrate various views of the at least one anchor and ADID according to some embodiments. Specifically, **Figure 2** is a second side view of an enlargement of the clip-device 150, the at least one anchor, and the ADID in combination attachment, where the at least one anchor is configured to be held by the clip-device, so as to, in some embodiments, enable packaging and/or transfer of the device.

As shown in **Figures 3A-B and 4****,** the at least one anchor can be a loop, which can include a metal or polymer (or a combination thereof), and multiple anchors of this configuration can be used to couple with the ADID 110 (and one or more of which used to anchor the ADID to tissue. In some embodiments, a single leg of the loop configuration can be considered the at least one anchor). In some embodiments, the at least one anchor includes the loop configuration (as shown in **Figures 2A-2D**), which can include a bulbous end or bulb 112a, which is coupled to the ADID 110 to an internal structure 110a therein (*see* cross-section, **Figure 3B**).

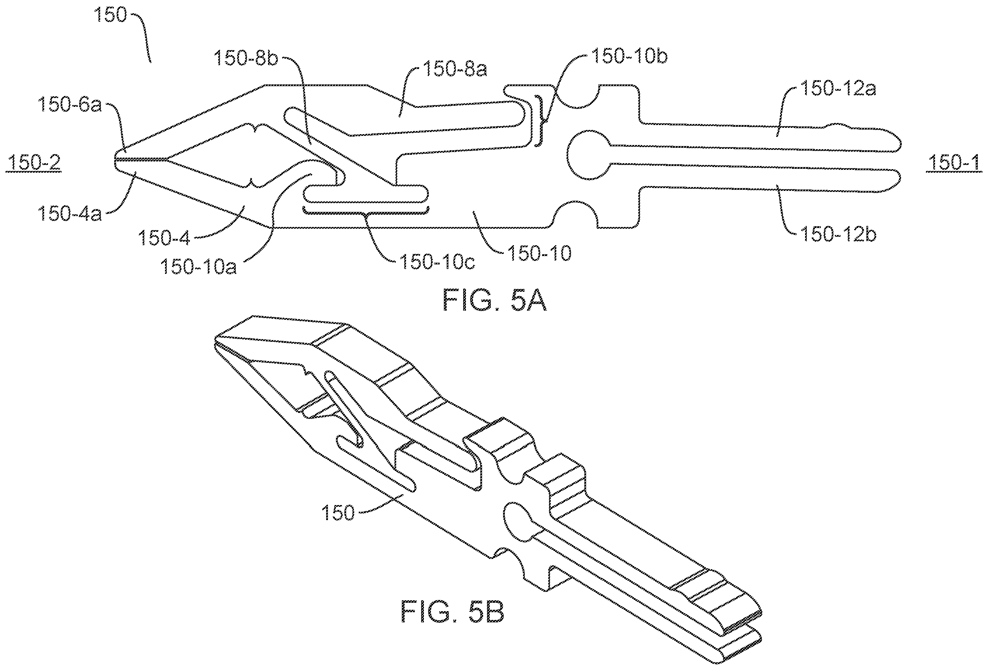

As shown in **Figures 5A-B****,** the at least one anchor can be configured to be held by the clip 150, where the clip can include a proximal end 150-1 and a distal end 150-2, a first jaw 150-4 having a corresponding first distal jaw end 150-4a, a second jaw 150-6 having a corresponding second distal jaw end 150-6a configured for movement relative to the first jaw end. A first jaw extension 150-10 may also be included, which can be integral with the first jaw 150-4, a second jaw extension 150-8 integral with the second jaw 150-6, and at least two proximal extensions 150-12a, 150-12b integral with the first jaw 150-4.

In some embodiments, in such this configuration, the first jaw extension 150-10 can include a first projection 150-10a, a first receiving area 150-10b, and a second receiving area 150-10c, the second extension 150-8 can include a flexible projection 150-8a, such that the first receiving area 150-10b can be configured to receive an end of the flexible projection 150-8a, the second receiving area 150-8c can be configured to receive the second jaw extension 150-8b and move therein, and/or the first distal end 150-4a of the first jaw 150-4 can be closed with respect to the second distal end 150-6a of the second jaw 150-6 in a resting position (for example).

In some embodiments, an analyte diffusive system is provided, and includes an analyte diffusive implantable device (ADID) which includes a cavity, a cell-scaffolding material (CSM) arranged within the cavity and configured to hold or otherwise retain a plurality of living cells, and a porous membrane at least partially enclosing the cavity, wherein at least a portion of the membrane is adhered to the CSM by a high durometer adhesive which creates a hemispheric seal at both ends of the membrane, and a filling port configured for connection to an end of the device. The port is configured to be in communication with the cavity and sealed thereto, to provide access to the cavity to enable cells to be received in the cavity, and as a polymer molded structure so as to allow an injection system to seal to thereto.

In such embodiments the micro-diameter tube 140 includes an inner micron-sized lumen configured to flow cells from an injection system into the cavity. Specifically, in some embodiments, the inner diameter of the associated lumen is between about 50-500 µm, and in some embodiments preferably between 100-150 µm. The tube includes a wall thickness and/or material configured to maintain a diameter geometry for delivery of cells through the tube and maintain the diameter geometry at a fill pressure of 0 - 150 psi (for example), and the tube 140 can be sealed to the port assembly with a first adhesive (or other means familiar to one of skill in the art). The tube 140 can be sealed within the CSM and membrane with a second adhesive (for example), having properties distinct from properties associated with the first adhesive (according to some embodiments).

A first end 140-2 of the tube 140 can be configured to connect to an end 110-2 of the ADID 110, a second end 140-4 of the tube is configured to connect to the port assembly 130, and cells are delivered from the injection system into the cavity of the ADID from the port assembly and through the tube 140. In some embodiments, the tube 140 is configured for preferential release from the first adhesive (e.g., holding the tube 140 to the port assembly 130), in which, a force required to preferentially release the sealed tube 140 from within the device, which in some embodiments, can require between 1-100 Newton-meters, or, in some embodiments, preferably between 30-35 Newton-meters.

The filling of cells within the cavity of the ADID 110 can be any one or more of: capillary action, positive displacement, vacuum, servo driven, peristaltic action, and combinations thereof.

In some embodiments, a cell transferring method for transferring cells into an analyte diffusive device is provided, or any device medical or otherwise (for that matter), and can include, in some embodiments for example, providing an analyte diffusive system according to any of the disclosed embodiments (e.g., those noted above), and sealing the tube with the ADID device and the port assembly 130. The method can further include, for example, transferring cells into the device via the port assembly 130 and tube 140 using, for example, an injection system. In some embodiments, the transferred cells are stored on/within the CSM within a cavity of the ADID 110, and removing at least one of the port assembly 130 and tube from the ADID 110.

Still further in some embodiments, an aseptic transferring method for transferring or otherwise moving an implantable device (or any device medical or otherwise, for that matter) is provided, the method includes providing an analyte diffusive implantable device (ADID), providing a clip, holding the device with the clip, where the clip attaches to the device either at one end of the device or via an anchor attached to the end of the device, and moving the device via the clip through automated manufacturing unit operations to fill the device.

In some embodiments, an aseptic method to maintain orientation of a device during storage and/or use for a surgical implant (or any device medical or otherwise, for that matter), the method includes providing an analyte diffusive system (or any device, medical or otherwise for that matter) including an analyte diffusive implantable device (ADID), providing a clip, holding the ADID with the clip, wherein the clip attaches to the ADID either at one end of the ADID or via an anchor attached to the end of the ADID and orients the ADID vertically and centrally in a packaging system containing cell nutrient media, and optionally aseptic transferring of the ADID from the packing system to a sclerotomy incision and facilitating positioning of the ADID and release of the ADID into the incision and vitreous cavity.

While various inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be an example and that the actual parameters, dimensions, materials, and configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims.

## Claims

1. A medical device system (100) comprising:
an analyte diffusive implantable device (ADID) (110) which comprises:
a cavity;
a cell-scaffolding material (CSM) arranged within the cavity and configured to hold or otherwise retain a plurality of living cells;
and
a porous membrane at least partially enclosing the cavity, wherein at least a portion of the membrane is adhered to the CSM by a high durometer adhesive which creates a hemispheric seal at both ends of the membrane;
a filling port (120) having:
docking means (130) at a first end, and
a micro-diameter tube (MDT) (140) extending from a second end;
at least one anchor (112) arranged on at least one end of the device;
and
a clip (150) for holding the anchor,
wherein the MDT is sealed at a distal end to the filling port (120) and at a proximal end to the cavity of the ADID (110), so as to establish an integral, cell-filling pathway there-within.

2. The system of claim 1, wherein the filling port comprises a temporary filling port.

3. The system of claim 1, wherein the anchor (112) is configured to affix the device to tissue.

4. The system of claim 1, wherein the clip (150) is configured to enable packaging and/or transfer of the device.

5. The system of claim 1, wherein the clip comprises:
a proximal end (150-1) and a distal end (150-2);
a first jaw (150-4) having a corresponding first distal jaw end (150-4a);
a second jaw (150-6) having a corresponding second distal jaw end (150-6a) configured for movement relative to the first jaw end (150-4a);
a first jaw extension (150-10) integral with the first jaw (150-4);
a second jaw extension (150-8) integral with the second jaw (150-6);
and
at least two proximal extensions (150-12a), (150-12b) integral with the first jaw (150-4);
wherein:
the first extension (150-12a) includes a first projection (150-10a), a first receiving area (150-10b) and a second receiving area (150-8c);
the second extension (150-12b) includes a flexible projection (150-8a);
the first receiving area (150-10b) being configured to receive an end of the flexible projection; the second receiving area (150-8c) being configured to receive the second jaw extension (150-8) and move therein; and
the first distal jaw end (150-4a) of the first jaw (150-4) is closed with respect to the second distal jaw end (150-6a) of the second jaw (150-6) in a resting position.

## Patentansprüche

1. Medizinisches Vorrichtungssystem (100), umfassend: eine implantierbare Analyt-Diffusionsvorrichtung (ADID) (110), die umfasst:
einen Hohlraum;
ein Zellgerüstmaterial (CSM), das in dem Hohlraum angeordnet und dazu ausgelegt ist, eine Vielzahl von lebenden Zellen zu halten oder anderweitig zu stützen; und
eine poröse Membran, die mindestens teilweise den Hohlraum umschließt, wobei mindestens ein Abschnitt der Membran an dem CSM haftet durch ein Klebemittel mit hohem Haftgrad, was eine halbkugelförmige Dichtung an beiden Enden der Membran erzeugt;
eine Einfüllöffnung (120) mit:
Andockmitteln (130) an einem ersten Ende, und
einer Röhre im Mikrodurchmesserbereich (MDT) (140), die sich von einem zweiten Ende erstreckt;
mindestens einem Anker (112), der an mindestens einem Ende der Vorrichtung angeordnet ist;
und
einer Klemme (150) zum Halten des Ankers,
wobei die MDT an einem distalen Ende gegenüber der Einfüllöffnung (120) und an einem proximalen Ende gegenüber der ADID (110) abgedichtet ist, um einen integralen zelleinfüllenden Weg dazwischen herzustellen.

2. System nach Anspruch 1, wobei die Einfüllöffnung eine temporäre Einfüllöffnung umfasst.

3. System nach Anspruch 1, wobei der Anker (112) dazu ausgelegt ist, die Vorrichtung am Gewebe zu fixieren.

4. System nach Anspruch 1, wobei die Klemme (150) dazu ausgelegt ist, das Verpacken und/oder Transportieren der Vorrichtung zu ermöglichen.

5. System nach Anspruch 1, wobei die Klemme umfasst:
ein proximales Ende (150-1) und ein distales Ende (150-2);
eine erste Backe (150-4) mit einem entsprechenden ersten distalen Backenende (150-4a);
eine zweite Backe (150-6) mit einem entsprechenden zweiten distalen Backenende (150-6a), das zur Bewegung bezogen auf das erste Backenende (150-4a) ausgelegt ist;
eine erste Backenverlängerung (150-10), einstückig mit der ersten Backe (150-4);
eine zweite Backenverlängerung (150-8), einstückig mit der zweiten Backe (150-6);
und
mindestens zwei proximale Verlängerungen (150-12a), (150-12b), einstückig mit der ersten Backe (150-4);
wobei:
die erste Verlängerung (150-12a) einen ersten Vorsprung (150-10a), eine erste Aufnahmefläche (150-10b) und eine zweite Aufnahmefläche (150-8c) aufweist;
die zweite Verlängerung (150-12b) einen flexiblen Vorsprung (150-8a) aufweist;
die erste Aufnahmefläche (150-10b) dazu ausgelegt ist, ein Ende des flexiblen Vorsprungs aufzunehmen; die zweite Aufnahmefläche (150-8c) dazu ausgelegt ist, die zweite Backenverlängerung (150-8) aufzunehmen und darin zu bewegen; und
das erste distale Backenende (150-4a) der ersten Backe (150-4) mit Bezug auf das zweite distale Backenende (150-6a) der zweiten Backe (150-6) in einer Ruheposition geschlossen ist.

## Revendications

1. Système de dispositif médical (100) comprenant :
un dispositif implantable de diffusion d'analyte (ADID) (110) qui comprend :
une cavité ;
un matériau d'échafaudage cellulaire (CSM) agencé à l'intérieur de la cavité et configuré pour contenir ou retenir une pluralité de cellules vivantes ;
et
une membrane poreuse entourant au moins partiellement la cavité, au moins une partie de la membrane étant collée au CSM par un adhésif à haut duromètre qui crée un joint hémisphérique aux deux extrémités de la membrane ;
un orifice de remplissage (120) comportant :
des moyens d'arrimage (130) à une première extrémité, et un tube de micro-diamètre (MDT) (140) s'étendant à partir d'une seconde extrémité ;
au moins un ancrage (112) agencé sur au moins une extrémité du dispositif ;
et
un clip (150) pour maintenir l'ancrage,
le MDT étant scellé au niveau d'une extrémité distale à l'orifice de remplissage (120) et au niveau d'une extrémité proximale à la cavité de l'ADID (110), de manière à établir une voie de remplissage cellulaire intégrée à l'intérieur de celle-ci.

2. Système selon la revendication 1, l'orifice de remplissage comprenant un orifice de remplissage temporaire.

3. Système selon la revendication 1, l'ancrage (112) étant configuré pour fixer le dispositif au tissu.

4. Système selon la revendication 1, le clip (150) étant configuré pour permettre l'emballage et/ou le transfert du dispositif.

5. Système selon la revendication 1, le clip comprenant :
une extrémité proximale (150-1) et une extrémité distale (150-2) ;
une première mâchoire (150-4) ayant une première extrémité de mâchoire distale correspondante (150-4a) ;
une seconde mâchoire (150-6) ayant une seconde extrémité de mâchoire distale correspondante (150-6a) configurée pour se déplacer par rapport à la première extrémité de mâchoire (150-4a) ;
une première extension de mâchoire (150-10) intégrée à la première mâchoire (150-4) ;
une seconde extension de mâchoire (150-8) intégrée à la seconde mâchoire (150-6) ;
et
au moins deux extensions proximales (150-12a), (150-12b) intégrées à la première mâchoire (150-4) ;
dans lequel :
la première extension (150-12a) comprend une première projection (150-10a), une première zone de réception (150-10b) et une seconde zone de réception (150-8c) ;
la seconde extension (150-12b) comprend une projection flexible (150-8a) ;
la première zone de réception (150-10b) est configurée pour recevoir une extrémité de la projection flexible ;
la seconde zone de réception (150-8c) est configurée pour recevoir la seconde extension de mâchoire (150-8) et s'y déplacer ; et
la première extrémité de mâchoire distale (150-4a) de la première mâchoire (150-4) est fermée par rapport à la seconde extrémité de mâchoire distale (150-6a) de la seconde mâchoire (150-6) en position de repos.
